# FASCICULE DE BREVET EUROPEEN

(11) **EP 0 438 948 B1**
(45) Date de publication et mention de la délivrance du brevet: **08.12.1993**
(21) Numéro de dépôt: 90403806.4
(22) Date de dépôt: 28.12.1990
(51) Int. Cl.: C07C 59/153, C07C 51/235

(54) **Procédé de préparation de l'acide glyoxylique par oxydation catalytique du glyoxal en milieu aqueux en présence de quantités catalytiques de platine**
Verfahren zur Herstellung von Glyoxylsäure durch die Oxydation des Glyoxals im wässerigen Milieu in Anwesenheit katalytischer Mengen von Platin
Process for preparing glyoxylic acid by catalytic oxidation of glyoxal in an aqueous medium in the presence of catalytic quantities of platinum

(30) Priorité: 23.01.1990 FR 9000734
(43) Date de publication de la demande: 31.07.1991
(73) Titulaire: SOCIETE FRANCAISE HOECHST, 92800 Puteaux (FR)
(72) Inventeur: De Mesantourne, Régine, F-79140 Cerizay (FR); Gallezot, Pierre, F-69001 Lyon (FR)
(74) Mandataire: Rinuy, Santarelli

(56) Documents cités:
- DE-A- 3 918 458
- FR-A- 2 069 836

## Description

La présente invention concerne un procédé de préparation de l'acide glyoxylique par oxydation catalytique du glyoxal en milieu aqueux en présence de quantités catalytiques de platine.

L'acide glyoxylique, qui est une matière première importante en synthèse organique, notamment pour l'obtention de la vanilline, est couramment obtenu par oxydation nitrique du glyoxal. Cette oxydation peut également être réalisée par voie électrochimique dans le compartiment anodique d'une cellule électrolytique.

Toutefois, ces procédés utilisent des réactifs d'oxydation indirecte nécessitant des purifications ultérieures se prêtant mal a une marche continue, et de ce fait, leur rentabilité n'est pas satisfaisante. Par ailleurs, il est connu d'obtenir de l'acide glyoxylique par oxydation catalytique de l'éthylène ou de l'acétaldéhyde mais la sélectivité de telles oxydations est médiocre.

On n'a encore jamais décrit une oxydation catalytique directe du glyoxal en acide glyoxylique par l'oxygène moléculaire. Or, il a maintenant été trouvé, ce qui constitue l'objet de la présente invention, un procédé de préparation de l'acide glyoxylique, caractérisé en ce que l'on soumet du glyoxal a une oxydation catalytique hétérogène, en milieu aqueux, par de l'oxygène en présence de quantités catalytiques de platine.

Dans des conditions avantageuses de mise en oeuvre de l'invention, le procédé ci-dessus décrit est réalisé à une température comprise entre 20 et 100°C. La réaction sera menée de préférence à une pression comprise entre la pression ambiante et 20 bars. La rapport molaire glyoxal/platine sera avantageusement compris entre 50 et 2000.

Lorsque le procédé de l'invention est mis en oeuvre à la pression atmosphérique, il est avantageux d'opérer avec un léger barbotage d'air ou mieux d'oxygène pur. Dans les autres cas, on travaille en vase clos en atmosphère d'air ou d'oxygène.

Avantageusement, le platine est déposé sur un support solide inerte et insoluble dans l'eau, de préférence solide, choisi notamment dans le groupe constitué par les graphites, les noirs de carbone et les charbons actifs. Le platine sera préférentiellement déposé à l'état de fines particules métalliques de taille homogène de 1,5 à 2 nm. Le support inerte choisi pourra être avantageusement un charbon actif à haute surface spécifique, supérieure à 1000 m² par gramme. On désigne habituellement par charbon actif, des corps très poreux obtenus par gazéification à la vapeur d'eau ou au dioxyde de carbone de substances carbonées non graphitables.

Le platine métallique pourra être déposé sur le support choisi selon les techniques habituelles soit par imprégnation avec une solution aqueuse d'acide hexachloroplatinique suivie d'une réduction, comme décrit par J.M. Dirkx et al, J. Catalysis, 1981, 67, 1-13, soit par échange cationique avec une solution aqueuse de chlorure de platine tétramine et réduction selon D. Richard et al, Preparation of Catalyst, Vol. IV, pages 71-81, Elsevier, Amsterdam, 1987. Avantageusement, on déposera entre 2 et 6 % en poids de platine sur le support. L'examen par microscopie électronique par transmission de ces supports permet de constater que la taille moyenne des particules métalliques de platine est approximativement de 1,5 à 2 nm.

Au fur et à mesure de l'oxydation du glyoxal en acide glyoxylique, le pH du milieu réactionnel diminue du fait de la forte acidité de l'acide glyoxylique formé. On dispose ainsi d'un moyen commode pour suivre l'avancement de la réaction. On peut, si désiré, travailler à pH constant en introduisant en continu dans le milieu réactionnel un agent alcalin hydrosoluble tel qu'un hydroxyde de métal alcalin comme l'hydroxyde de sodium ou l'hydroxyde de potassium pour salifier l'acide glyoxylique formé.

Les exemples suivants illustrent la présente invention sans toutefois la limiter.

### Exemple 1

### Préparation du catalyseur

On agite du charbon actif de surface spécifique 1450 m²/g pendant 24 heures à 50°C, dans 10 volumes d'acide chlorhydrique 2N. La suspension est alors filtrée et le précipité recueilli est lavé à l'eau jusqu'à élimination complète des ions chlorure. Ce précipité est ensuite soumis à une combustion partielle pendant 3 heures à 500°C sous un courant d'air de 120 ml/min. Après refroidissement à la température ambiante, on obtient un charbon présentant une surface spécifique de 1800 m²/g.

On dissout, en atmosphère inerte, 1 g (1,93 mmole) d'acide hexachloroplatinique cristallisé avec 6 molécules d'eau dans 20 g d'eau distillée, puis cette solution est introduite en atmosphère inerte dans une suspension agitée de 7,2 g du charbon obtenu précédemment et la suspension obtenue est agitée à la température ambiante pendant 330 minutes en atmosphère inerte. On refroidit alors la suspension à 0°C et à cette température, on introduit 19,5 g d'une solution aqueuse de formaldéhyde à 37 %, soit 0,24 mole de formaldéhyde puis, 10 minutes plus tard, 11,6 g d'une solution aqueuse d'hydroxyde de potassium à 30 % en poids, soit 0,21 mode d'hydroxyde de potassium. La suspension obtenue est ensuite agitée durant 16 heures à la température ambiante puis elle est filtrée. Le précipité est lavé à l'eau jusqu'à neutralité des eaux de lavage et il est ensuite séché sous pression réduite à 100°C pendant 13 heures. On obtient ainsi un catalyseur contenant 4,2 % en poids de platine métallique sous forme de fines particules métalliques de taille homogène de 1,5 à 2 nm.

### Exemple 2

On agite à la vitesse de 400 tours par minute, durant 110 minutes à 38 ± 2°C, sous un barbotage d'air de 100 ml par minute, en maintenant le pH du milieu réactionnel à 7,7 par addition en continu d'une solution aqueuse diluée d'hydroxyde de potassium, la suspension suivante constituée par :
- 304,36 g d'une solution aqueuse de glyoxal à 0,573 % en poids, soit 1,745 g (30 mmoles) de glyoxal,
- 0,750 g du catalyseur préparé à l'exemple 1 contenant 4,2 % en poids de platine, soit 31,5 mg (0,161 mmole) de platine métallique.

On obtient ainsi une suspension contenant :
- 2,47 g (22 mmoles) de glyoxylate de potassium,
- 0,75 g (4,5 mmoles) d'oxalate de potassium,
- 0,13 g (2,25 mmoles) de glyoxal non transformé.

Le rendement de l'oxydation est de 73 % de la théorie calculée par rapport au glyoxal mis en oeuvre et la sélectivité est de 79,3 %.

## Revendications

1. Procédé de préparation de l'acide glyoxylique, caractérisé en ce que l'on soumet du glyoxal à une oxydation catalytique hétérogène en milieu aqueux par de l'oxygène en présence de quantités catalytiques de platine.

2. Procédé selon la revendication 1, caractérisé en ce qu'il est mis en oeuvre à une température comprise entre 20 et 100°C.

3. Procédé selon l'une quelconque des revendications 1 ou 2, caractérisé en ce qu'il est mis en oeuvre à une pression comprise entre la pression ambiante et 20 bars.

4. Procédé selon l'une quelconque des revendications 1 à 3, caractérisé en ce que le rapport molaire glyoxal sur platine est compris entre 50 et 2000.

5. Procédé selon l'une quelconque des revendications 1 à 4, caractérisé en ce que le platine est déposé sur un support solide inerte et insoluble dans l'eau.

6. Procédé selon la revendication 5, caractérisé en ce que le support inerte est un charbon actif de surface spécifique supérieure à 1000 m²/g.

## Claims

1. Process for the preparation of glyoxylic acid, characterised in that glyoxal is subjected to a heterogeneous catalytic oxidation in an aqueous medium by oxygen in the presence of catalytic quantities of platinum.

2. Process according to Claim 1, characterised in that it is carried out at a temperature between 20 and 100°C.

3. Process according to either one of Claims 1 or 2, characterised in that it is carried out at a pressure between ambient pressure and 20 bars.

4. Process according to any one of Claims 1 to 3, characterised in that the molar ratio of glyoxal to platinum is between 50 and 2000.

5. Process according to any one of Claims 1 to 4, characterised in that the platinum is deposited on an inert solid carrier which is insoluble in water.

6. Process according to Claim 5, characterised in that the inert carrier is an activated charcoal with a specific surface area greater than 1000 m²/g.

## Patentansprüche

1. Verfahren zur Herstellung von Glyoxylsäure, dadurch gekennzeichnet, daß man Glyoxal einer heterogenen katalytischen Oxidation in wässrigem Medium durch Sauerstoff in Anwesenheit von katalytischen Mengen von Platin unterwirft.

2. Verfahren nach Anspruch 1, dadurch gekennzeichnet, daß es bei einer Temperatur zwischen 20 und 100° C durchgeführt wird.

3. Verfahren nach einem der Ansprüche 1 oder 2, dadurch gekennzeichnet, daß es bei einem Druck zwischen Umgebungsdruck und 20 bar durchgeführt wird.

4. Verfahren nach einem der Ansprüche 1 bis 3, dadurch gekennzeichnet, daß das Molverhältnis von Glyoxal zu Platin zwischen 50 und 2000 liegt.

5. Verfahren nach einem der Ansprüche 1 bis 4, dadurch gekennzeichnet, daß das Platin auf einem festen, inerten und in Wasser unlöslichen Träger abgelagert ist.

6. Verfahren nach Anspruch 5, dadurch gekennzeichnet, daß der inerte Träger eine Aktivkohle mit einer spezifischen Oberfläche oberhalb von 1000 m²/g ist.
